# EUROPEAN PATENT APPLICATION

(11) **EP 2 401 972 A2**
(43) Date of publication of application: **04.01.2012**
(21) Application number: 11007906.8
(22) Date of filing: 30.04.2003
(51) Int. Cl.: A61B 17/08

(54) **Endoluminal surgical clips for gastroesophageal reflux disease (GERD)**

(30) Priority: 20.05.2002 US 151529
(62) Divisional of application: 03738884.0
(71) Applicant: Ethicon Endo-Surgery, Inc., Cincinnati, OH 45242 (US)
(72) Inventor: Sixto, Rober, Jr., Miami FL 33156 (US); Kortenbach, Juergen A., Miami Springs FL 33166 (US)
(74) Representative: Müller - Hoffmann & Partner

(57) **Abstract**

Surgical clips (910), which are particularly useful in the transoral invagination and fundoplication of the stomach to the esophagus, are disclosed. The clips include first and second arms (912 & 914) joined by a bridge to form a substantially U-shape, and which are provided with a first structure (931/932) adapted to prevent a movement of the clip in a direction perpendicular to a longitudinal axis of the clip after the clip is applied to tissue. In addition, the clips preferably also include a second structure (922/924) adapted to prevent rotation of the clip about the longitudinal axis of the clip after the clip is applied to tissue.

## Description

### BACKGROUND OF THE INVENTION

### 1. Field of the Invention

The invention relates to endoscopic surgical procedures and instruments. More particularly, the invention relates to surgical clips which are particularly useful in the transoral invagination and fundoplication of the stomach to the esophagus.

### 2. State of the Art

Gastroesophageal fundoplication is a procedure for the treatment of gastroesophageal reflux disease (GERD), a condition in which gastric acids are regurgitated into the esophagus resulting in one or more of esophagitis, intractable vomiting, asthma, and aspiration pneumonia. The fundoplication procedure involves wrapping the fundus of the stomach around the lower end of the esophagus and fastening it in place. Traditionally, this procedure is accomplished via open surgery with the use of sutures to secure the plicated fundus of the stomach around the esophagus without penetrating (incising) the stomach. Although traditional fundoplication involves plicating the fundus and the esophagus, as used herein the term includes plicating the fundus to itself near the esophagus.

U.S. Patent Number 5,403,326 to Harrison et al. discloses a method of performing endoscopic fundoplication using surgical staples or two-part surgical fasteners. The procedure disclosed by Harrison et al. involves performing two percutaneous endoscopic gastrotomies (incisions through the skin into the stomach) and the installation of two ports through which a stapler, an endoscope, and an esophageal manipulator (invagination device) are inserted. Under view of the endoscope, the esophageal manipulator is used to pull the interior of the esophagus into the stomach. When the esophagus is in position, with the fundus of the stomach plicated, the stapler is moved into position around the lower end of the esophagus and the plicated fundus is stapled to the esophagus. The process is repeated at different axial and rotary positions until the desired fundoplication is achieved. While, the procedure disclosed by Harrison et al. is a vast improvement over open surgery, it is still relatively invasive requiring two incisions through the stomach.

U.S. Patent Number 5,571,116 to Bolanos et al. discloses a non-invasive treatment of gastroesophageal reflux disease which utilizes a remotely operable invagination device and a remotely operable surgical stapler, both of which are inserted transorally through the esophagus. According to the methods disclosed by Bolanos et al., the invagination device is inserted first and is used to clamp the gastroesophageal junction. The device is then moved distally, pulling the clamped gastroesophageal junction into the stomach, thereby invaginating the junction and involuting the surrounding fundic wall. The stapler is then inserted transorally and delivered to the invaginated junction where it is used to staple the fundic wall.

Bolanos et al. disclose several different invagination devices and several different staplers. Generally, each of the staplers disclosed by Bolanos et al. has an elongate body and a spring biased anvil which is rotatable approximately 15 degrees away from the body in order to locate the invaginated gastroesophageal junction between the body and the anvil. The body contains a staple cartridge holding a plurality of staples, and a staple firing knife. Each of the invagination devices disclosed by Bolanos et al. has a jaw member which is rotatable at least 45 degrees and in some cases more than 90 degrees to an open position for grasping the gastroesophageal junction. One of the chief disadvantages of the methods and apparatus disclosed by Bolanos et al. is that the stapler and the invagination device must both be present in the esophagus at the same time. With some of the embodiments disclosed, the presence of both instruments is significantly challenged by the size of the esophagus. In addition, the actuating mechanism of the device disclosed by Bolanos et al. is awkward. In particular, the stapler anvil is biased to the open position, and it is not clear whether or not the stapler anvil can be locked in a closed position without continuously holding down a lever. In addition, it appears that the staple firing trigger can be inadvertently operated before the anvil is in the closed position. This would result in inadvertent ejection of staples into the stomach or the esophagus of the patient.

U.S. Patent Number 6,086,600 to Kortenbach discloses an endoscopic surgical instrument including a flexible tube, a grasping and fastening end effector coupled to the distal end of the tube, and a manual actuator coupled to the proximal end of the tube. The manual actuator is coupled to the end effector by a plurality of flexible cables which extend through the tube. The tube contains a lumen for receiving a manipulable endoscope and the end effector includes a passage for the distal end of the endoscope. The end effector has a store for a plurality of male fastener parts, a store for a plurality of female fastener parts, a rotatable grasper, a rotatable fastener head for aligning a female fastener part and a male fastener part with tissues therebetween, and a firing member for pressing a male fastener part through tissues grasped by the grasper and into a female fastener part. According to a stated preferred embodiment, the overall diameters of the flexible tube and the end effector (when rotated to the open position) do not exceed approximately 20 mm so that the instrument may be delivered transorally to the fundus of the stomach.

While transoral invagination and fundoplication apparatus and procedures have improved over the years, it is still difficult to deliver and manipulate the necessary apparatus transorally. The primary reason for the difficulty is that the overall diameter, or more accurately the cross sectional area, of the equipment is too large. Notwithstanding Kortenbach's reference to 20mm, most of the equipment in use today is at least 24mm in diameter. Moreover, even if the equipment could be reduced to 20mm in diameter (314 mm² cross sectional area), it would still be difficult to manipulate. Those skilled in the art will appreciate that larger instruments are less pliable and that the invagination and fundoplication procedure requires that the instruments turn nearly 180 degrees. Moreover, it will be appreciated that large instruments obscure the endoscopic view of the surgical site.

Still other issues which need to be addressed in this procedure include the need to suitably grasp the fundus before plication so that all layers of the fundus are plicated. Preferably, plication damages the deep muscle and serosa inciting an inflammatory response causing adhesions to occurs during healing.

### 3. Co-owned Technology

Previously incorporated application Serial Number 09/730,911, filed December 6, 2000, entitled "Methods and Apparatus for the Treatment of Gastric Ulcers", discloses a surgical tool which is delivered to a surgical site over an endoscope rather than through the working lumen of an endoscope.

Co-owned provisional application Serial Number 60/292,419, filed May 21, 2001, entitled "Methods and Apparatus for On-Endoscope Instruments Having End Effectors and Combinations of On-Endoscope and Through-Endoscope Instruments", discloses many tools and procedures including an on-scope grasper assembly having grasping jaws, and a throughscope clip applier having jaws adapted to close about tissue and apply a clip over and/or through the tissue. In operation, the grasper jaws may grab and hold tissue, e.g., the fundus of the stomach or esophageal tissue, while the jaws of the clip applier surround a portion of the tissue held by the grasper jaws and apply a clip thereover.

Previously incorporated application Serial Number 09/891,775, filed June 25, 2001, entitled "Surgical Clip", discloses a surgical clip having a U-shaped configuration with first and second arms, and a bridge portion therebetween. The first arm is provided with a tip preferably having a catch, and the second arm extends into a deformable retainer having a tissue-piercing end and preferably also a hook. During application, tissue is clamped, and the clip is forced over the clamped tissue and the retainer of the second arm is bent and may be pierced through the tissue. The retainer is toward and around or adjacent the tip of the first arm preferably until the hook is engaged about the catch to secure the clip to the tissue and prevent the clip and tissue from separating. The clip is provided with structure that facilitates the stacking of a plurality of clips in a clip chamber of a clip applier.

Previously incorporated application Serial Number 09/931,528, filed August 16, 2001, entitled "Methods and Apparatus for Delivering a Medical Instrument Over an Endoscope while the Endoscope is in a Body Lumen", discloses methods and apparatus for delivering a medical instrument over the exterior of an endoscope while the endoscope is installed in the patient's body in order to allow the use of instruments which are too large to fit through the lumina of an endoscope.

The previously incorporated simultaneously filed application entitled "Flexible Surgical Clip Applier", discloses a surgical clip applier having a pair of clip applying jaws at the distal end of an outer coil, a set of pull wires extending through the outer coil and coupled to the jaws, and a push wire extending through the outer coil. A clip chamber is provided in the distal end of the coil. A clip pusher is provided at a distal end of the push wire, and adapted to advance a clip into the jaws. The jaws include clamping surfaces which operate to compress tissue between the jaws when the jaws are closed, channels in which a distalmost clip rides when the jaws are closed and the pusher is advanced thereby causing the distalmost clip to be pushed over the tissue, and distal anvil portions which operate to bend a portion of the distalmost clip to facilitate its retention on the clamped tissue. The clip applier is capable of providing a pushing force far in excess of a perceived possible maximum of the 200 grams (0.44 lbs) published in the art. One embodiment of the device of the invention provides a pushing force in excess of 2267 grams (51bs).

### SUMMARY OF THE INVENTION

It is therefore an object of the invention to provide methods and apparatus for transoral invagination and fundoplication.

It is also an object of the invention to provide an apparatus for transoral invagination and fundoplication which is easy to manipulate.

It is another object of the invention to provide an apparatus for transoral invagination and fundoplication which has a relatively small cross-sectional area.

It is still another object of the invention to provide methods and apparatus for fundoplication which combine the relative advantages of staples and two-part fasteners, i.e. the small size of a staple and the greater integrity of a two-part fastener.

It is yet another object of the invention to provide methods and apparatus for transoral invagination and fundoplication which damages tissue such that adhesion occurs during healing.

In accord with these objects which will be discussed in detail below, the methods of the invention include delivering a grasper, a clip applier, and an endoscope transorally to the site of fundoplication; grasping the fundus with the grasper (or similar device, e.g. corkscrew) and pulling it into the jaws of the clip applier; closing the jaws of the clip applier over the fundus and applying a clip to the fundus. The method is repeated at different locations until the desired fundoplication is achieved. The apparatus of the invention includes a clip applier having sharp toothed jaws for grasping and damaging the fundus prior to applying the clip. The clip applier has an overall diameter of less than 7mm and may be delivered through a 7mm sleeve which attaches to a 12mm endoscope having a lumen through which the grasper is delivered. The overall cross-sectional area of the apparatus is therefore approximately 152 mm² as compared to the 314mm² of the prior art devices. Alternatively, the clip applier and the grasper may be delivered through an endoscope having two 6mm lumina.

According to a presently preferred embodiment, the clip applier jaws are coupled to a pull wire via a linkage which increases the mechanical advantage and thus permits greater grasping force.

A plurality of clip designs are provided. Some embodiments include a pair of arms coupled by a bridge and a single locking retainer. Other embodiments include dual parallel coiled retainers. According to one embodiment, the clip has two detachable retainers which are installed in the fundus and the clip arms and bridge are removed.

Additional objects and advantages of the invention will become apparent to those skilled in the art upon reference to the detailed description taken in conjunction with the provided figures.

### BRIEF DESCRIPTION OF THE DRAWINGS

Figure 1 is a side elevational view of a clip applier according to the invention;
Figure 2 is a side elevational view of a first embodiment of the distal end of the clip applier with the jaws in the closed position;
Figure 3 is a side elevational view of a first embodiment of the distal end of the clip applier with the jaws in the open position;
Figure 4 is a broken isometric view of a first embodiment of the distal end of the clip applier with one jaw removed;
Figure 5 is a broken isometric view of a second embodiment of the distal end of the clip applier with a clip of the type shown in Figures 19 and 20;
Figure 6 is an isometric view of a single jaw of the second embodiment of the distal end of the clip applier;
Figure 7 is a proximal end view of the jaw of Figure 6;
Figure 8 is a proximal end view of the two jaws of a second embodiment of the distal end of the clip applier in the closed position with the lower jaw shaded for clarity;
Figure 9 is a broken isometric view of a third embodiment of the distal end of the clip applier suitable for use with a clip of the type shown in Figures 17 and 18 or 24;
Figures 10-14 are schematic views illustrating a method according to the invention;
Figure 15 is a diagram illustrating the comparative cross-section of the instruments used in the method illustrated in Figures 5-10 and a typical prior art instrument;
Figure 16 is a cross-sectional view of a dual lumen endoscope which can be used in performing the methods of the invention;
Figure 17 is a side elevational view of a first embodiment of a clip according to the invention prior to application;
Figure 18 is a side elevational view of the clip of Figure 17 after application;
Figure 19 is a side elevational view of a second embodiment of a clip according to the invention prior to application;
Figure 20 is a side elevational view of the clip of Figure 19 after application;
Figure 21 is a side elevational view of a third embodiment of a clip according to the invention prior to assembly;
Figure 22 is a side elevational view of the clip of Figure 21 assembled prior to application;
Figure 23 is a side elevational view of the applied portion of the clip of Figures 17 and 18;
Figure 24 is a view similar to Figure 23 of an alternate third embodiment of the applied portion of a clip according to the invention;
Fig. 25 is a side elevational view of a fourth embodiment of a clip according to the invention prior to application;
Fig. 26 is a side elevational view of the clip of Fig. 25, shown in an applied configuration;
Fig. 27 is a side elevational view of a fifth embodiment of a clip according to the invention prior to application;
Fig. 28 is a top view of the clip of Fig. 27 shown in a configuration prior to application;
Fig. 29 is a side elevational view of the clip of Fig. 27, shown in an applied configuration;
Fig. 30 is a side elevational view of a sixth embodiment of a clip according to the invention prior to application;
Fig. 31 is a side elevational view of the clip of Fig. 30, shown in an applied configuration;
Fig. 32 is a side elevational view of a seventh embodiment of a clip according to the invention prior to application;
Fig. 33 is a side elevational view of the clip of Fig. 32, shown in an applied configuration;
Fig. 34 is a side elevational view of an eighth embodiment of a clip according to the invention prior to application;
Fig. 35 is a top view of the eighth embodiment of a clip according to the invention prior to application;
Fig. 36 is a side elevational view of the clip of Fig. 34, shown in an applied configuration; and
Fig. 37 is a top view of the eighth embodiment of a clip according to the invention shown in an applied configuration.

### DETAILED DESCRIPTION OF THE PREFERRED EMBODIMENTS

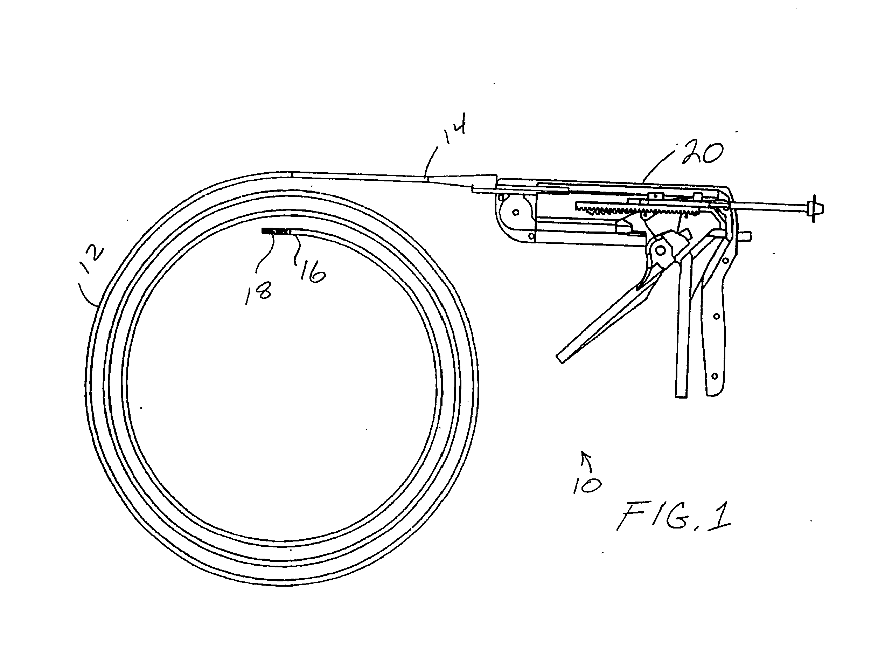

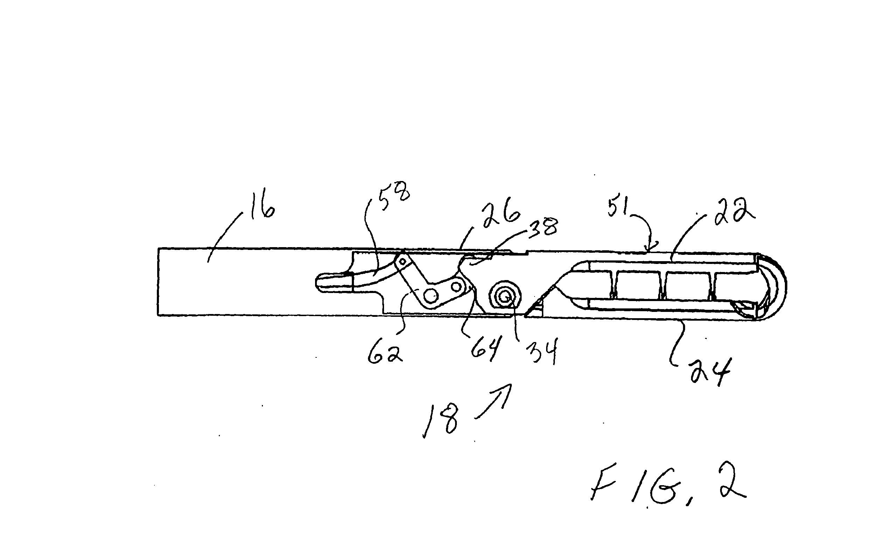

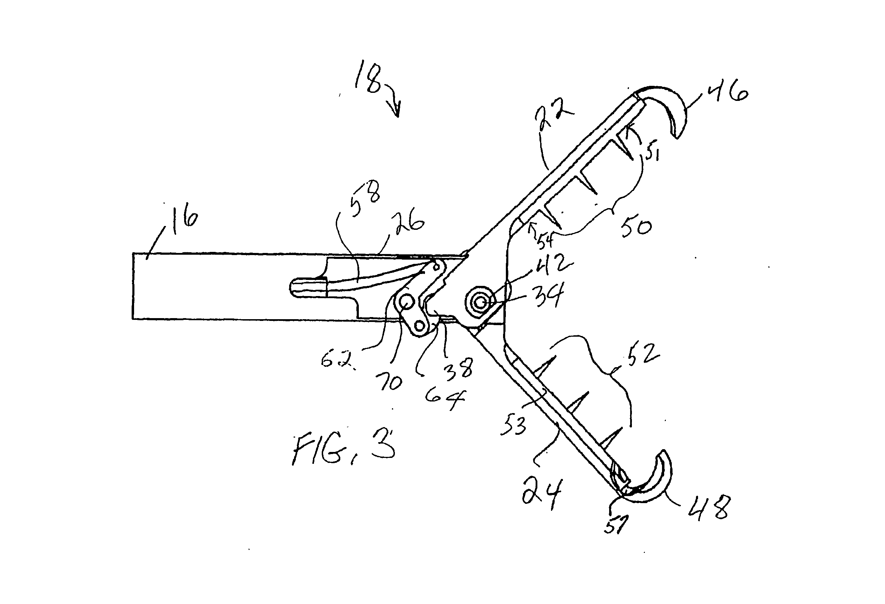

Referring now to Figure 1, a clip applier 10 according to the invention generally includes a flexible wound outer coil 12 having a proximal end 14 and a distal end 16. An end effector assembly 18 is coupled to the distal end 16 of the coil 12 and an actuator assembly 20 is coupled to the proximal end 14 of the coil 12. A plurality of pull/push wires 58, 60 (shown and described below with reference to Figures 2-4) extend through the coil 12 and couple the end effector assembly 18 to the actuator assembly 20. The clip applier 10 is similar to the clip applier described in detail in previously incorporated co-owned application Serial Number 10/010,096, filed Dec. 12, 2001. However, in this application, the end effector assembly 18 is designed specifically for fundoplication using a clip significantly larger than that used in the clip applier of the aforesaid co-owned application.

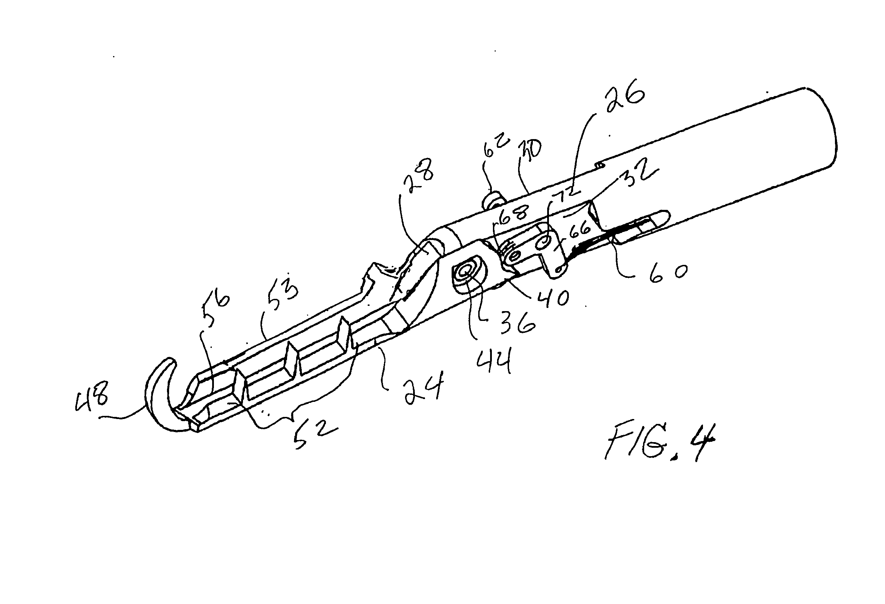

Figures 2-4 illustrate the details of the end effector assembly 18 according to a first embodiment of the invention. The end effector assembly 18 includes a pair of jaws 22, 24 which are rotatably coupled to a clevis 26. In particular, the clevis 26 has a central channel 28 (seen best in Figure 4) which is defined by clevis arms 30, 32. Although the term "clevis" is used because of its general acceptance in the art of endoscopic instruments, the "clevis" 26 is preferably covered on top and bottom so that the only exit from the channel 28 is at the distal end. The jaw 22 is rotatably coupled to the clevis arm 30 by an axle 34 and the jaw 24 is rotatably coupled to the clevis arm 32 by an axle 36. The axles 34 and 36 are dimensioned such that they do not significantly obscure the channel 28.

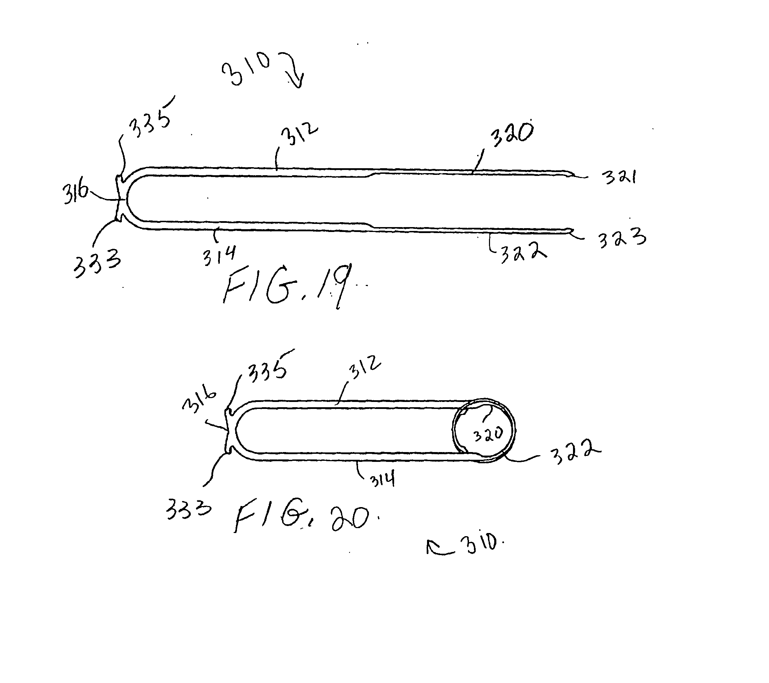

The jaws 22, 24 are substantially identical. Each jaw 22, 24 includes a proximal tang 3 8, 40, a mounting bore 42, 44, a distal hook shaped anvil 46, 48 and a plurality of medial teeth 50, 52. As seen best in Figure 4, the medial teeth 50, 52 are arranged on one side of the jaw and a short wall 51, 53 is arranged on the opposite side of the jaw to define a groove (or guiding channel) 54, 56. The grooves 54, 56 meet the anvils 46, 48 each of which has a helical surface. The interior (proximal) helical surfaces of the anvils act to bend the clip retainers as described below with reference to Figures 19-24.

The proximal tang 38, 40 of each jaw is coupled to a respective pull/push wire 58, 60 via two links 62, 64 and 66, 68. The links 62, 66 are substantially L-shaped and are rotatably coupled near their elbow to the clevis arms 30, 32 by axles 70, 72 which do not significantly obscure the channel 28 between the clevis arms. One end of the link 62, 66 is coupled to the pull/push wire 58, 60 and the other end of the link 62, 66 is rotatably coupled to one end of the link 64, 68. The other end of the link 64, 68 is rotatably coupled to the tang 38, 40. The combined coupling of each jaw 22, 24 to each pull/push wire 58, 60 forms a linkage which amplifies the force from the pull/push wires to the jaws. In particular, as the jaws close, the mechanical advantage increases.

The proximal ends of the pull/push wires 58, 60 are coupled to the actuator assembly (20 in Figure 1) as described in previously incorporated co-owned application Serial Number 10/010,096, entitled "Flexible Surgical Clip Applier", and filed Dec. 12, 2001.

A clip pusher (not shown) disposed in the interior of the coil is coupled to a push wire (not shown) which is coupled to the actuator assembly as described in previously incorporated co-owned application Serial Number 10/010,096, filed Dec. 12, 2001. Unlike the previously incorporated co-owned application, the jaws of the instant clip applier are significantly longer and designed for use with clips approximately 17-20mm long (after the clip is applied) as compared to the 5-7mm clips shown in the previously incorporated co-owned application.

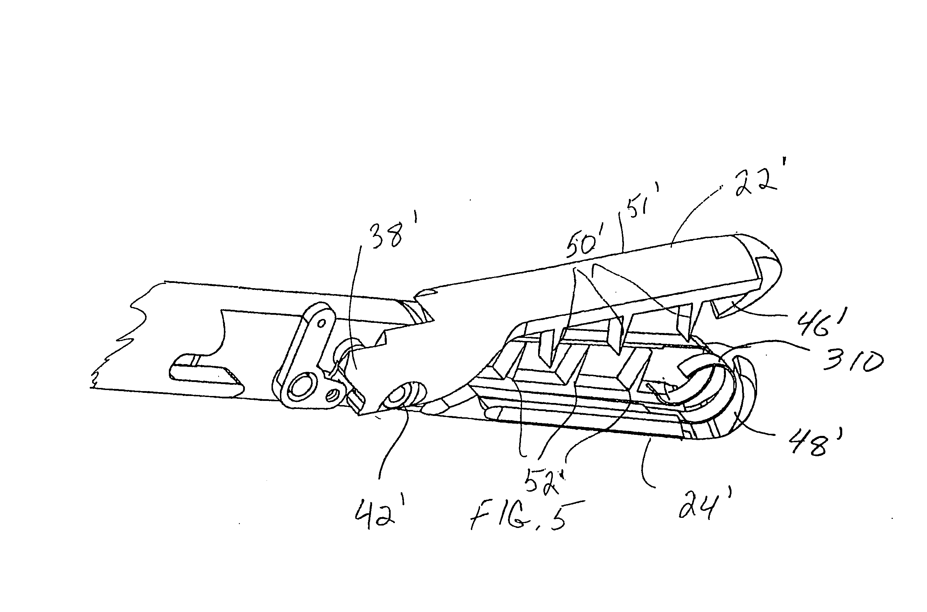

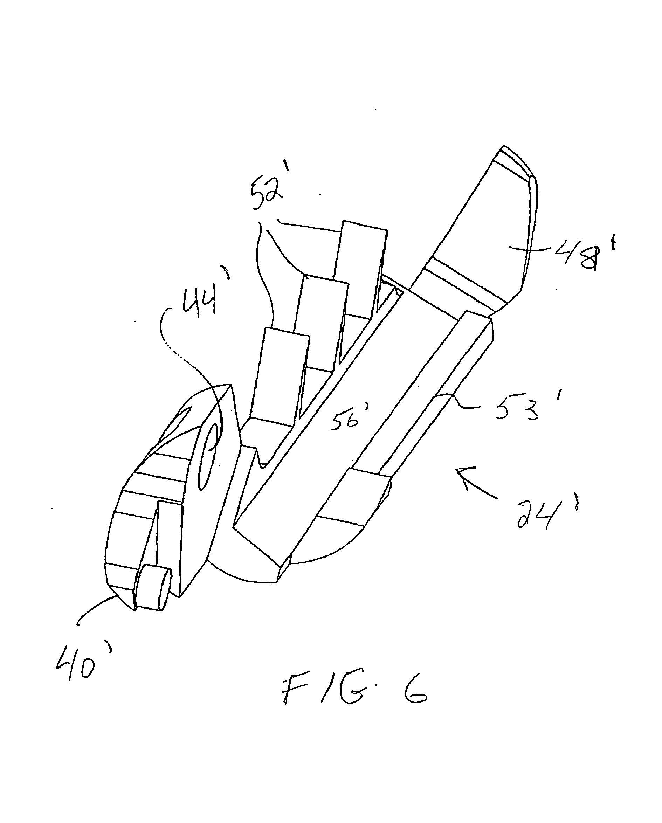

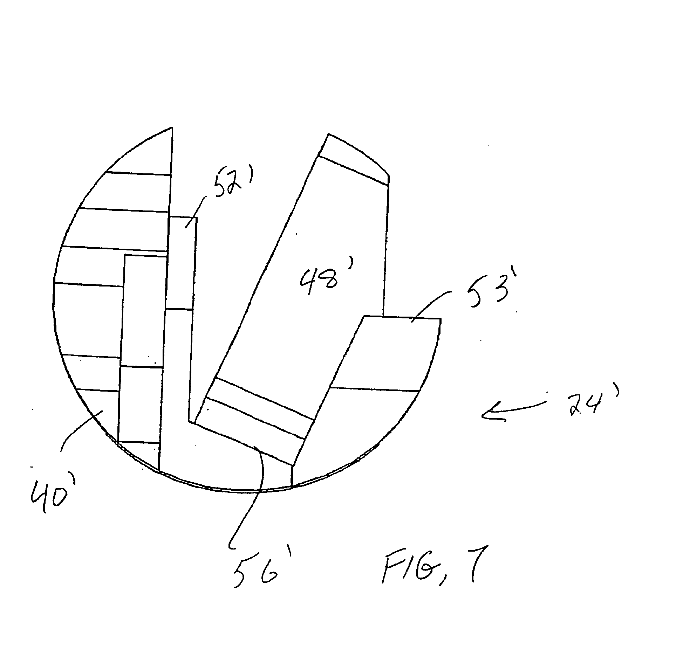

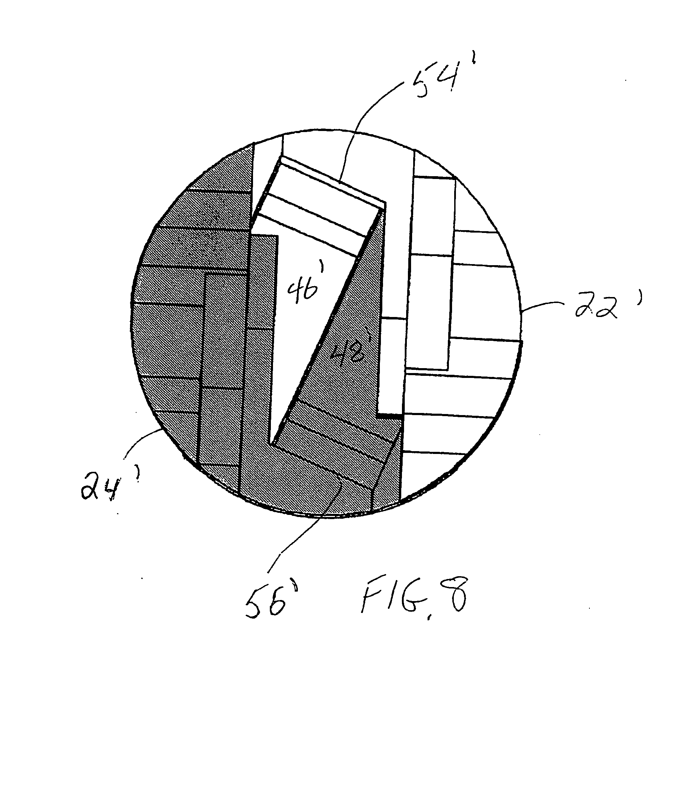

Turning now to Figures 5-8, a second embodiment of the jaws 22', 24' is illustrated. The jaws 22', 24' are substantially identical to each other and are designed for use with any of the clips illustrated in Figures 19-24. Each jaw 22', 24' includes a proximal tang 38', 40', a mounting bore 42', 44', a distal hook shaped anvil 46', 48' and a plurality of medial teeth 50', 52'. The medial teeth 50', 52' are arranged on one side of the jaw and a short wall 51', 53' is arranged on the opposite side of the jaw to define a groove (or guiding channel) 54', 56'. The grooves 54', 56' meet the interior surfaces of the anvils 46', 48' which curve about a single axis. The interior surfaces of the anvils act to bend the clip retainers as described below with reference to Figures 19-24 and as shown by the clip 310 in Figure 5. According to this embodiment, as seen best in Figures 6-8, the guiding channels 54', 56' and the anvils 46', 48' are angled relative to the vertical axis of the jaw 22', 24'. This angle causes the clip to twist as it is pushed through the jaws so that the ends of the clip are offset as shown in Figure 5, for example. According to the presently preferred embodiment, the guiding channels 54', 56' and the anvils 46', 48' are angled approximately 22° relative to the vertical axis of the jaw 22', 24'. According to a method of the invention, clips for use with this embodiment of the jaws are pre-bent in the bridge area to facilitate movement through the angled channels.

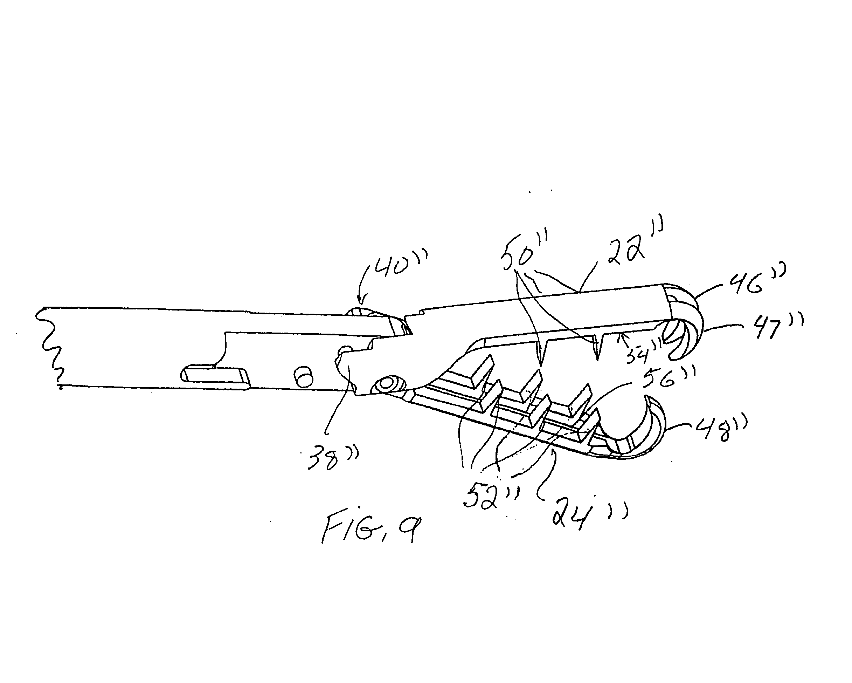

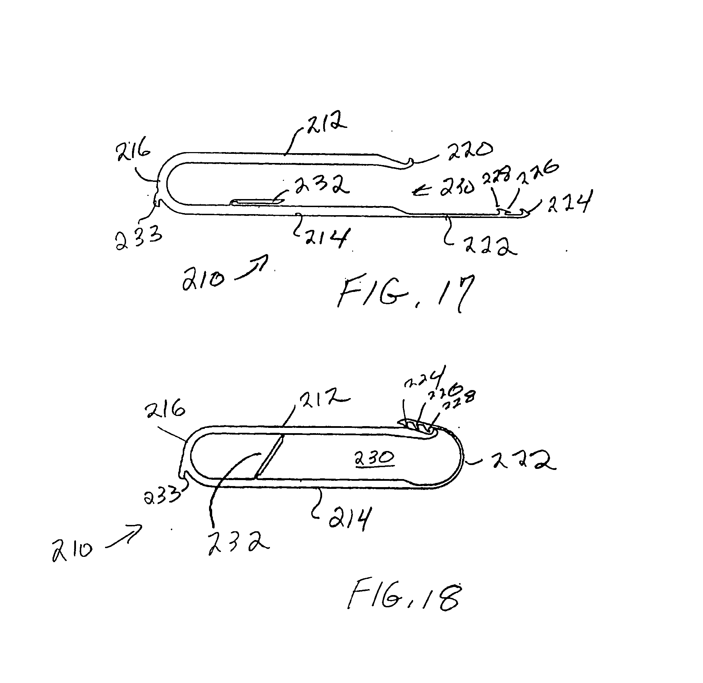

Referring now to Figure 9, a third embodiment of the jaws 22", 24" is illustrated. The jaws 22", 24" are not identical to each other and are designed for use with clips of the type illustrated in Figures 17-18. Each jaw 22", 24" includes a proximal tang 38", 40" and a mounting bore 42", 44". One jaw 22" terminates with two spaced apart distal hooks 46", 47" and has two rows of medial teeth 50". The other jaw 24" terminates with a single distal hook shaped anvil 48" and has two rows of medial teeth 52". The medial teeth 50", 52" are arranged on both sides of the jaw and a groove (or guiding channel) 54", 56" lies between the rows of teeth. The groove 54" terminates with an undercut well (not shown) as described in co-owned Serial Number 10/010,096, whereas the groove 56" continues on to the interior of the anvil 48" which has a surface which curves about a single axis. Those skilled in the art will appreciate that when the jaws are closed, the anvil 48" will reside between the hooks 46" and 47" and the teeth 50" will be interleaved with the teeth 52". The interior surface of the anvil 48" bends the clip retainer as described below with reference to Figures 17-18 and as shown and described in previously incorporated co-owned applications Serial Number 09/891,775, and Serial Number 10/010,096.

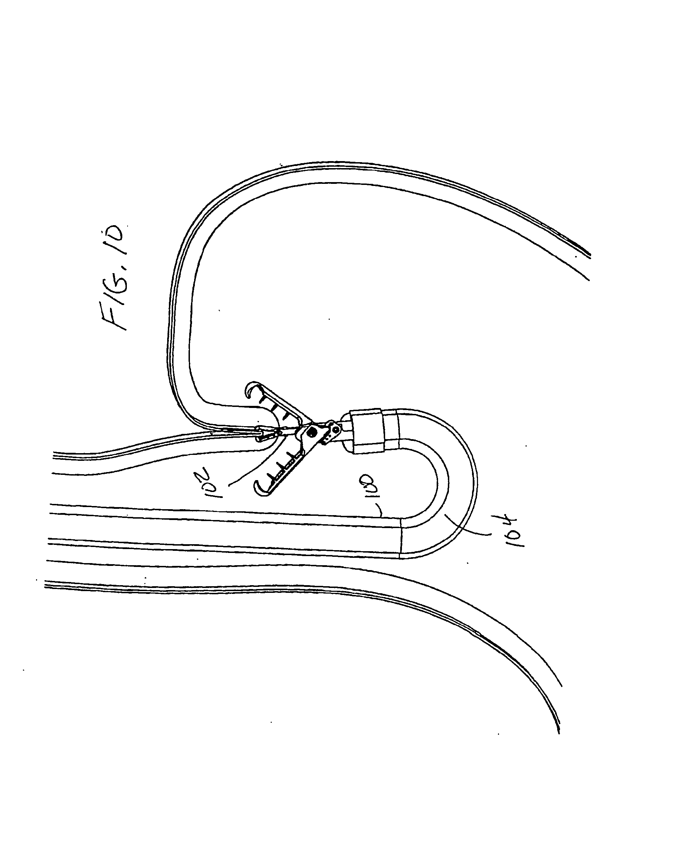

Turning now to Figures 10-14, a method of using the clip applier of the invention is illustrated in context with an existing endoscope 100 having a single lumen through which a small grasper 102 is supplied and an external working channel 104 which is attached to the scope 100 and through which the clip applier is delivered. The external working channel 104 is preferably one of the type described in previously incorporated application Serial Number 09/931,528, filed August 16, 2001, entitled "Methods and Apparatus for Delivering a Medical Instrument Over an Endoscope while the Endoscope is in a Body Lumen".

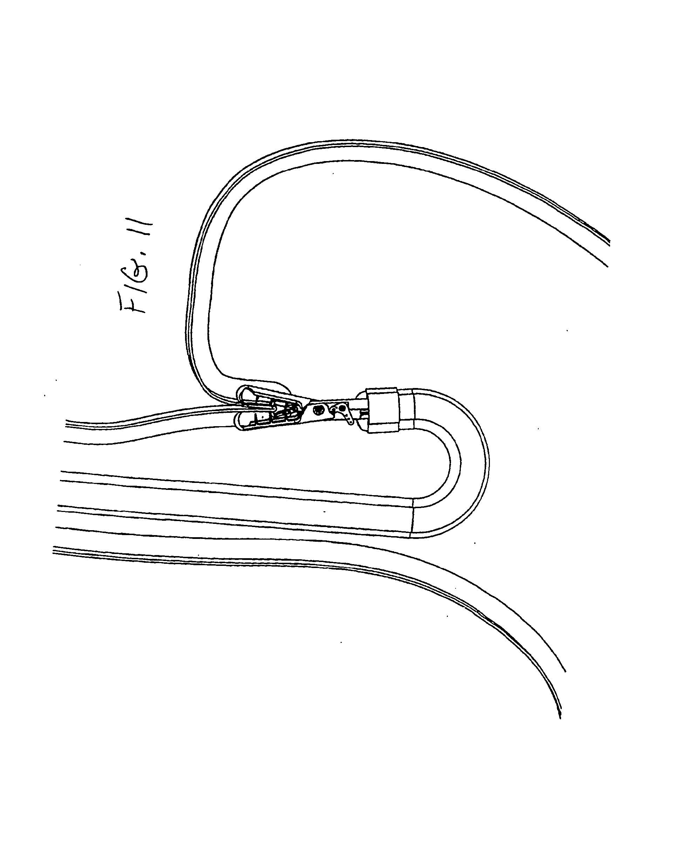

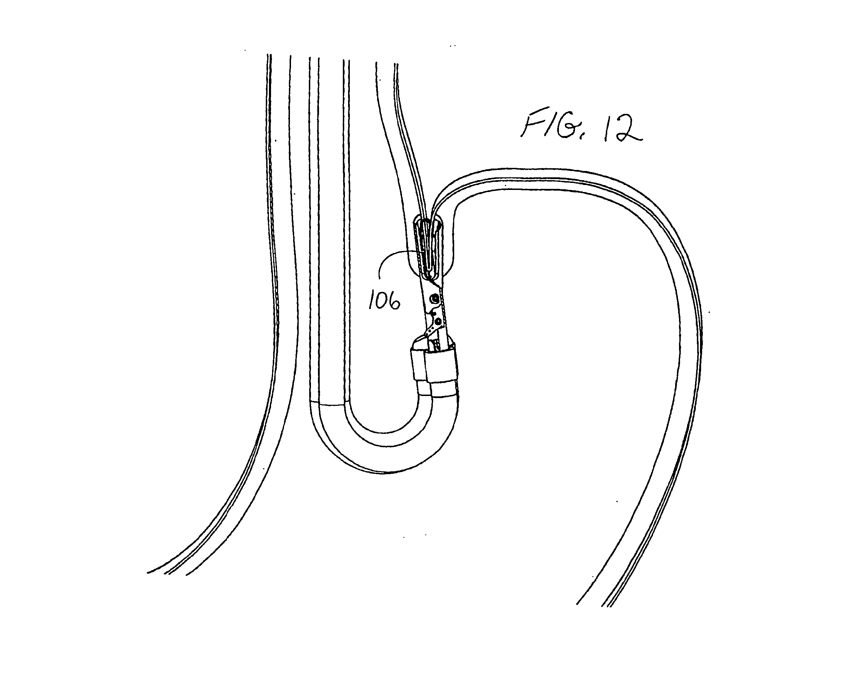

According to a method of the invention, after the endoscope assembly is delivered transorally to the procedural site, as shown in Figure 10, the fundus is grasped by the graspers and pulled in between the open jaws of the clip applier. The jaws of the clip applier are then closed onto the invaginated fundus as shown in Figure 11. As the jaws are closed the medial teeth of the jaws puncture the invaginated fundus as shown in Figures 11 and 12. When the jaws are completely closed (or closed as much as possible), they are preferably locked, the grasper is optionally released, and the clip pusher is activated to push forward a clip 106 as shown in Figure 12 and as described in co-owned Serial. No. 10/010,096, filed Dec. 12, 2001.

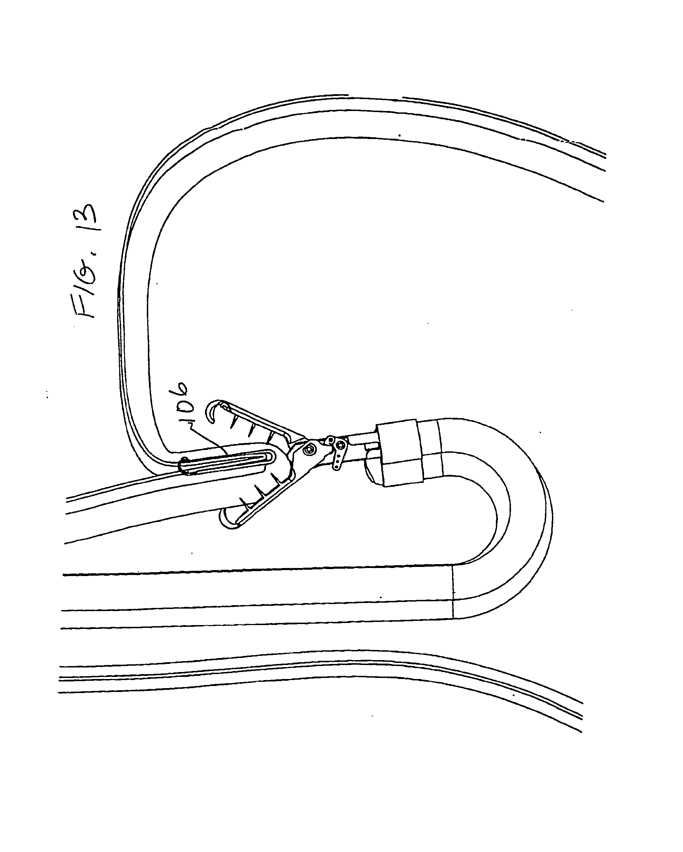

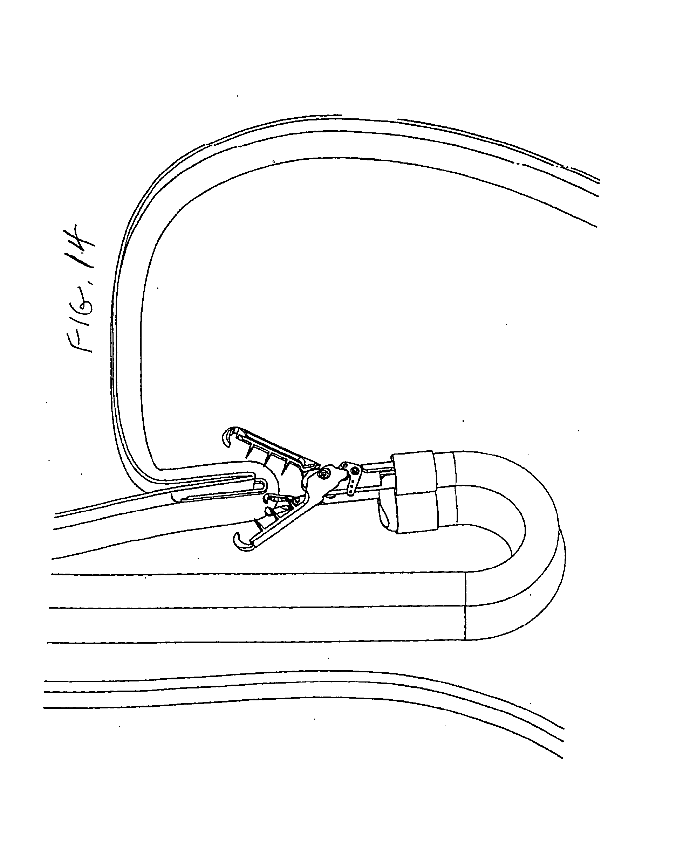

After the clip 106 is applied, the jaws of the clip applier are opened as shown in Figure 13 and the clip 106 remains in place and plicates the fundus. Depending on the location of the clip and the nature of the patient's condition, a single clip may be sufficient. If other clips are deemed desirable by the practitioner, the clip applier is removed and re-loaded with another clip. After re-delivering the clip applier, the procedure may be repeated at another location as shown in Figure 14. Given the size of the clips of the invention, anywhere from 1-4 clips will typically be used.

According to one aspect of the invention, the medial teeth on the jaws of the clip applier are long enough and sharp enough to damage the fundus sufficiently such that when the fundus heals adhesion occurs, binding the plicated fundus to the extent that the clip may no longer be needed. Thus, preferably, the teeth are long enough to pierce all layers of the fundus.

From the foregoing, those skilled in the art will appreciate that the methods of the invention may be performed with different types of graspers. In particular, alternative grasping devices such as a "cork screw" grasper can be used in conjunction with the clip applier of the invention to perform the methods of the invention.

It will also be appreciated that the clip applier of the invention may be attached to an endoscope in other ways as described in previously incorporated application Serial Number 09/931,528, filed August 16, 2001, entitled "Methods and Apparatus for Delivering a Medical Instrument Over an Endoscope while the Endoscope is in a Body Lumen".

As mentioned above, the clip applier of the invention has an outside diameter of approximately 6mm. As shown in Figures 10-14, the clip applier is used in conjunction with an endoscope having an outside diameter of approximately 12mm. To accommodate the clip applier, an exterior working channel having an exterior diameter of approximately 7mm is optionally coupled to the endoscope as described in the previously incorporated co-owned applications Serial Numbers 09/931,528 and 60/292,419.

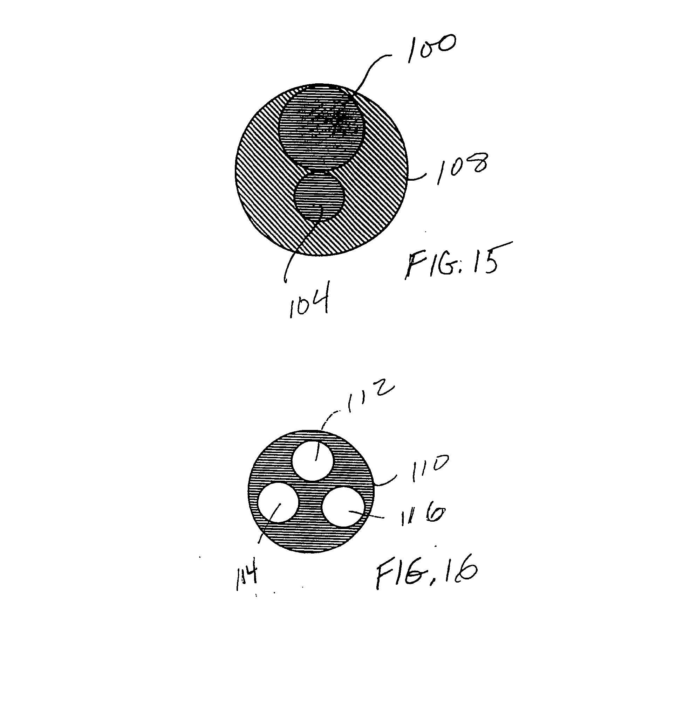

Figure 15 is a scale representation of the cross-sectional area of the 12mm endoscope 100 with the attached external 7mm working channel 104, shown in horizontal shading. The cross sectional area of a prior art device 108 having an exterior diameter of approximately 24mm is shown in diagonal shading. From Figure 15, it will be appreciated that the methods and apparatus of the invention allow for a substantially smaller device which is more easily delivered transorally and which is more easily manipulated. The overall cross-sectional area of the apparatus of the invention is approximately 152mm² as compared to the 314mm² of the prior art devices.

As mentioned, the clip applier of the invention may also be used with a dual lumen endoscope. Figure 16 is a scale representation of a dual lumen endoscope 110 having an optical lumen 112 and two 6mm working lumina 114, 116. As compared to the device 108 in Figure 15, the endoscope 110 has a substantially smaller cross-sectional area than the prior art device.

The clips used by the clip applier of the invention are substantially longer than the clips described in the previously incorporated co-owned applications, Serial Number 09/891,775 and the simultaneously filed application, which are approximately 7 mm in length and adequate for general surgical applications. The retainer portion of the clips of the present invention are substantially longer in order to assure that all of the layers of the fundus are punctured.

Turning now to Figures 17 and 18, a first embodiment of a surgical clip 210 according to the invention includes first and second arms 212, 214, respectively, and a bridge portion 216 therebetween such that the arms and bridge portion are in a generally U-shaped configuration. The first arm 12 is provided with an end catch 220, and the second arm 214 extends (or transitions) into a deformable retainer 222 having a tissue piercing tip 224 and a plurality of catch engagements, e.g. 226, 228. The arms define an open space 230 between them. The clip 210 is preferably made from a unitary piece of titanium, titanium alloy, stainless steel, tantalum, platinum, other high Z (substantially radiopaque) materials, nickel-titanium alloy, martensitic alloy, or plastic, although other suitable biocompatible materials may be used. The first and second arms 212, 214, as well as the bridge portion 216 are relatively stiff and not plastically deformable within the limits of force applied to the arms during use, while the retainer 222 is relatively easily plastically deformable by the clip applier.

Referring now to Figures 2-4 and 17-18, when the clip 210 is pushed forward in the clip applier with the jaws 22, 24 of the clip applier closed, the retainer 222 is bent across the opening 230 between the first and second arms 212, 214 and into engagement with the end catch 220 of the first arm 212 as shown in Figure 18. The anvil formed by the grooves on the interior of the hooks 46, 48 of the clip applier jaws guide the bending of the retainer 222 causing it to puncture the fundus and couple to the end catch 220.

The clip 210 shown in Figures 17 and 18 is provided with an optional bendable barb 232 which provides a secondary stabilizing fixation point which helps keep the clip from rotating. As the clip is pushed forward over the fundus, tissue catches the barb 232 and bends it as shown in Figure 18.

The clip 210 is also provided with an ear 233 on the bridge 216. The ear is used by the pushing mechanism (not shown) to grasp the end of the clip when it is loaded into the clip applier.

A second embodiment of a clip 310 according to the invention is shown in Figures 19 and 20. The clip 310 has two arms 312, 314 connected by a bridge 316. Both arms terminate in retainers 320, 322, each having a sharp end 321, 323. The clip 310 is also provided with a pair of ears 333, 335 on the bridge 316. The ears are used by the pushing mechanism (not shown) to grasp the end of the clip when it is loaded into the clip applier. This embodiment is intended for use with a clip applier having hooks with interior grooves which diverge, or which are in parallel planes. With reference to Figures 2-4 and 15-16, when the clip 310 is pushed forward, the retainer 320 is bent by the groove inside the hook 46 and the retainer 322 is bent by the groove inside the hook 48 to the configuration shown in Figure 20. From Figure 20, it will be appreciated that each retainer punctures the fundus twice substantially forming a circular fastener. Thus, it will also be appreciated that the retainers 320, 322 are significantly longer than the retainer 222 shown in Figures 17 and 18 and preferably are of a length at least π times the distance between the arms 312, 314. Insofar as the retainers 320, 322 each form a complete fastener, the function of the arms 312, 314 and the bridge 316 may be considered redundant.

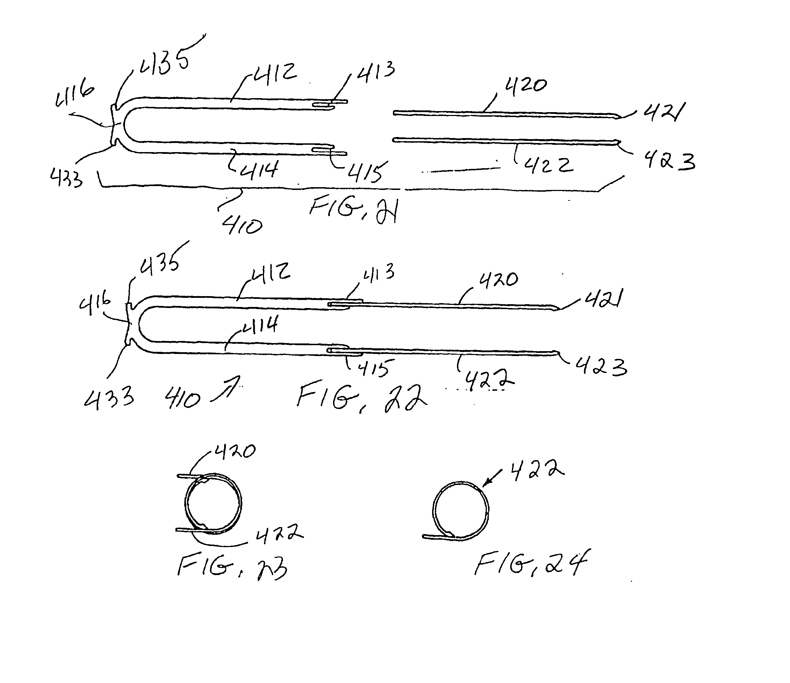

Figures 21-23 illustrate a third embodiment of a clip 410 according to the invention. The clip 410 is similar to the clip 310 (with similar reference numerals increased by 100 referring to similar parts) except that the retainers 420, 422 are removable from the arms 412, 414. The arms 412, 414 terminate in female couplings 413, 415 which receive ends of the retainers 420, 422 in a slight interference fit. The clip 410 is also provided with a pair of ears 433, 435 on the bridge 416. The ears are used by the pushing mechanism (not shown) to grasp the end of the clip when it is loaded into the clip applier. The ears 433, 435 may also be used as a structure by which to engage a clip applied over tissue, e.g., with a snare, to pull and remove the clip from the tissue. The clip 410 is applied to the fundus in substantially the same way as described above with reference to the clip 310. However, after the retainers 420, 422 are bent by the anvils and the jaws are opened, the clip 410 is not released from the clip applier and the retainers are separated from the arms 412, 414. The resulting fastener formed by the retainers 420, 422 is shown in Figure 23. This is actually two substantially parallel "b" shaped fasteners. Thus, it may only be necessary to apply a single retainer as shown in Figure 24, for example.

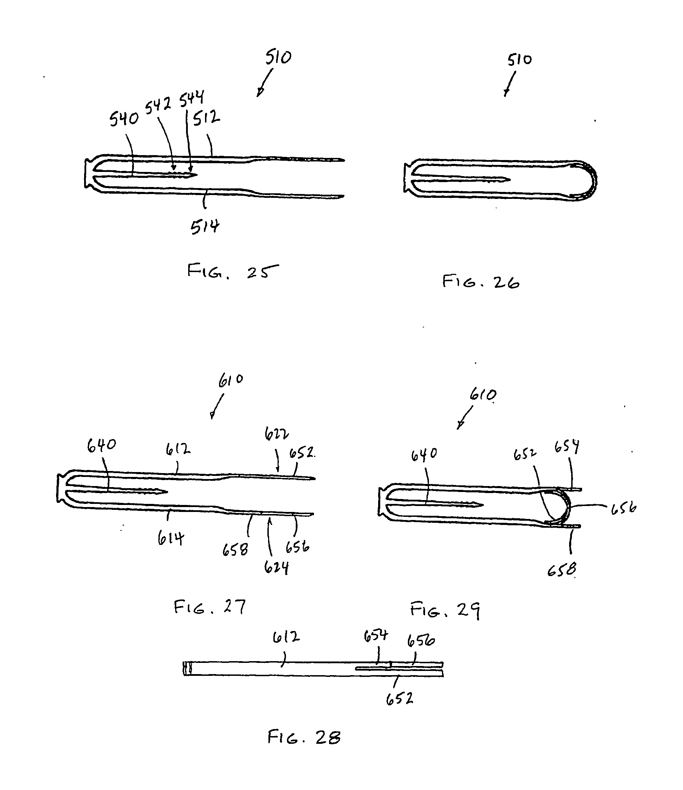

Figures 25 and 26 illustrate a fourth embodiment of a clip 510. The clip 510 is similar to the clip 310 (with similar reference numerals increased by 200 referring to similar parts) with the addition of a central prong 540 extending between the arms 512, 514, such that the arms and prong are together configured in an 'E'-shape. The prong 540 preferably includes a set of tissue irritators (or irregularities) 542 as well as a barbed, sharp tip 544. Alternatively, the prong can have a radiused or flat tip, yet nevertheless be of a dimension adapted to pierce tissue. In addition, the prong need not include the irritators. However, the tissue irritators, when provided, scratch and disturb the serosa causing desirable adhesions. The prong 540, when pierced through tissue of the fundus as the clip is applied to the fundus, prevents undesirable movement of the clip 510 in a direction perpendicular to the axis of the prong. The clip 510 is applied to the fundus in substantially the same way as described above with reference to the clip 310.

Figures 27 through 29 illustrate a fifth embodiment of a clip 610. The clip 610 is similar to the clip 510 (with similar reference numerals increased by 100 referring to similar parts), with the addition that the retainers 622, 624 of arms 612, 614 are bifurcated. A first portion 652 of the bifurcated retainer 622 is bent by an anvil of the jaw assembly of the clip applier, while the second shorter portion 654 preferably remains substantially straight. Likewise, a first portion 656 of the bifurcated retainer 624 is bent by an anvil of the jaw assembly of the clip applier, while the second shorter portion 658 preferably remains substantially straight. When the clip is applied to the fundus and the straight portions 654, 658 are pierced through the tissue in a direction substantially parallel to the prong 640, the straight portions 654, 658 prevent undesirable rotational movement of the clip 610 about an axis extending through the prong of the clip. The clip 610 is applied to the fundus in substantially the same way as described above with reference to clip 310.

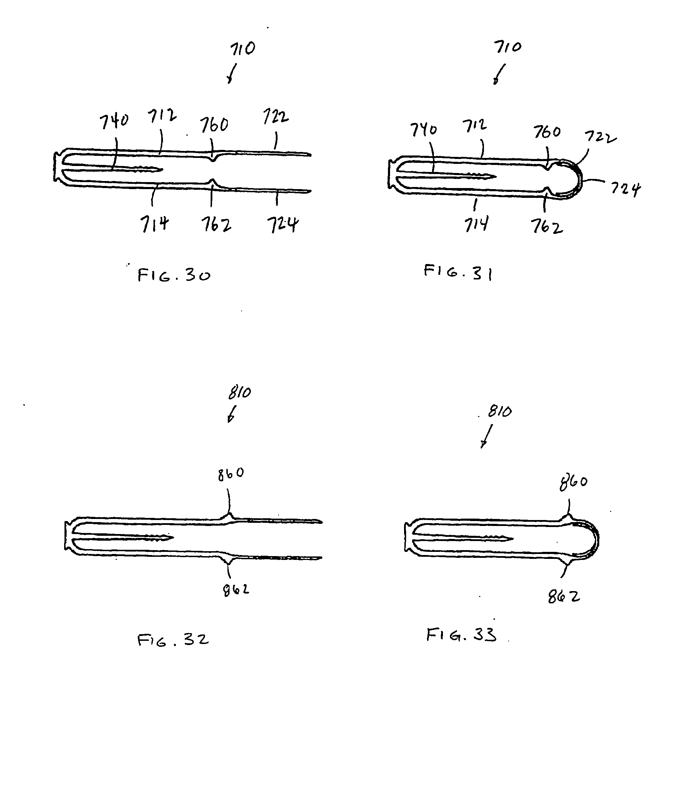

Figures 30 and 31 illustrate a sixth embodiment of a clip 710. The clip 710 is similar to the clip 510 (with similar reference numerals increased by 200 referring to similar parts), with the addition that the arms 712, 714 each include an inwardly directed projection 760, 762 adjacent the retainers 722, 724. The projections 760, 762 provide a non-slip function to the clip. As such, the projections 760, 762 prevent undesirable rotational movement of the clip 710 about an axis extending through the prong of the clip, as well as assist in preventing movement of the clip 710 in a direction perpendicular to the axis of the prong. The clip 710 is applied to the fundus in substantially the same way as described above with reference to clip 310.

Figures 32 and 33 illustrate a seventh embodiment of a clip 810. The clip 810 is similar to the clip 710, except that the projections 860, 862 are outwardly directed.

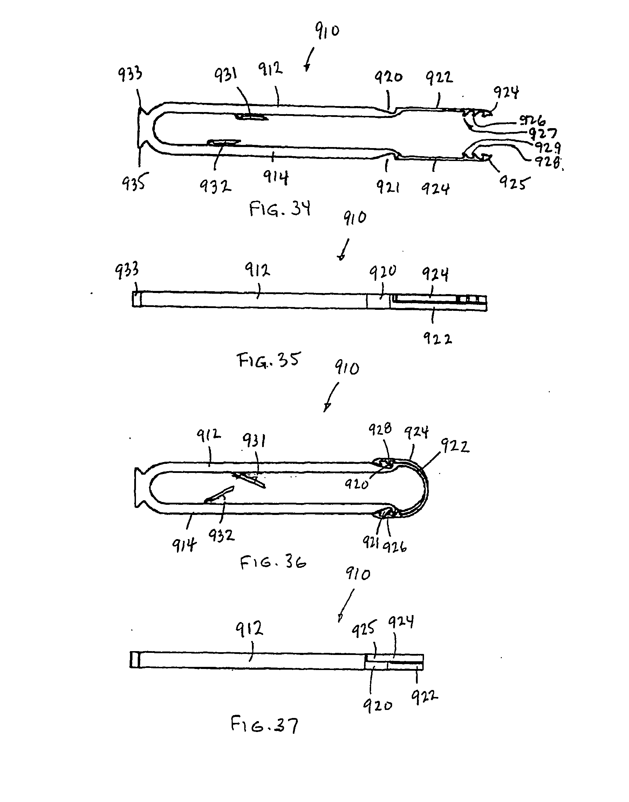

Figures 34 and 35 illustrate an eighth embodiment of a clip 910. The clip 910 is similar to the clip 210 (with similar reference numerals increased by 700 referring to similar parts). Each arm 912, 914 of the clip 910 is provided with a catch 920, 921 adjacent an end thereof, and a preferably half-width deformable retainer 922, 924; i.e., the retainers are preferably approximately half the width of the arms 912, 914. Retainer 922 is provided with a piercing tip 924 at its end, and plurality of catch engagements or barbs 926, 927, and retainer 924 is likewise provided with a piercing tip 925 at its end, and plurality of catch engagements or barbs 928, 929. The half-width retainers 922, 924 are laterally offset relative to each other. As such, referring to Figures 36 and 37, when the retainers 922, 924 are deformed by the jaws of the clip applier, the retainers are each bent preferably 180° and one of the catch engagements on retainer 922, e.g., catch engagement 926, preferably comes around to engage within the catch 921, and one of the catch engagements on retainer 924, e.g., catch engagement 928, preferably comes around to engage within the catch 920. In addition, clip 910 preferably includes two barbs 931, 932, one at the inside of each arm 912, 914, which are bendable upon being driven into tissue and which provide secondary stabilizing fixation points which helps keep the clip 910 from rotating. Furthermore, clip 910 preferably also includes ears 933, 935 which permit engagement of the clip for advancement by a clip applier and for removal.

With respect to the above embodiment, it is appreciated that the retainers do not each need to be half the width of the arms, but only that the combined widths of the retainers preferably be less than the width of the arms; i.e., one retainer may have a width of one-third the width of an arm, while the other retainer may have a width of up to two-thirds the width of an arm.

In all of the clip embodiments, the preferred range of dimensions for the clips is as follows. The length of the arms of the clip is preferably approximately 15 to 40 mm, and the length of the retainer is preferably an additional approximately 2 to 10 mm. The width of the clip is preferably approximately 2 to 6 mm. The length of the prong, when provided, is preferably approximately 2 to 20 mm. It is, however, appreciated that the clips may be provided with other relative dimensions.

There have been described and illustrated herein several embodiments of methods and apparatus for the endoluminal treatment of gastroesophageal reflux disease. While particular embodiments of the invention have been described, it is not intended that the invention be limited thereto, as it is intended that the invention be as broad in scope as the art will allow and that the specification be read likewise. In addition, while one manner of fundoplication has been described in which the stomach is attached to the esophagus, it is also recognized that one portion of the stomach can be attached to another portion of the stomach to reduce compliance of the lower esophageal sphincter, and the clips described above can be used for such a procedure. Also, it is appreciated that various features of the several clips can be combined with features of other clips, and the not all features shown with respect to each clip is required. For example, projections on the retainers may be included where the clip does not include a central prong. It will therefore be appreciated by those skilled in the art that yet other modifications could be made to the provided invention without deviating from its spirit and scope as claimed.

Support for the claims and further embodiments are defined in the following itemized list:
1. A surgical clip, comprising:
   a) a first arm;
   b) a second arm;
   c) a bridge connecting said first and second arms such that said first and second arms and said bridge are configured in a U-shape; and
   d) a first structure adapted to prevent a movement of said clip in a direction perpendicular to a longitudinal axis of said clip after said clip is applied to said tissue.
2. A surgical clip according to item 1, wherein:
   said first structure is located between said first and second arms.
3. A surgical clip according to item 2, wherein:
   said first structure is a prong extending parallel to and between said first and second arms,
   wherein said first and second arms, said prong and said bridge are together configured in an E-shape.
4. A surgical clip according to item 3, wherein:
   said prong is provided with at least one tissue irritator.
5. A surgical clip according to item 3, wherein:
   said prong has an end provided with a barb.
6. A surgical clip according to item 1, wherein:
   said prong has one of a sharp tip and a radiused tip.
7. A surgical clip according to item 1, further comprising:
   e) a second structure adapted to prevent rotation of said clip about said longitudinal axis of said clip after said clip is applied to said tissue.
8. A surgical clip according to item 1, further comprising:
   e) at least one deformable retainer extending from one of said arms, wherein said arms are relatively stiffer than said at least one deformable retainer.
9. A surgical clip according to item 7, further comprising:
   f) at least one deformable retainer extending from one of said arms, wherein said arms are relatively stiffer than said at least one deformable retainer, wherein said at least one deformable retainer is bifurcated into first and second portions, and said second portion of said at least one deformable retainer is said second structure adapted to prevent rotation.
10. A surgical clip according to item 7, wherein:
   said second structure includes one of inwardly-directed and outwardly-directed projection on at least one of said arms.
11. A surgical clip for insertion into tissue, comprising:
   a) a first arm;
   b) a second arm;
   c) a bridge connecting said first and second arms such that said first and second arms extend in substantially a same direction; and
   d) a first structure adapted to prevent rotation of said clip about a longitudinal axis of said clip after said clip is applied to said tissue.
12. A surgical clip according to item 11, further comprising:
   e) at least one deformable retainer extending from one of said arms, wherein said arms are relatively stiffer than said at least one deformable retainer.
13. A surgical clip according to item 12, wherein:
   said at least one deformable retainer is bifurcated into first and second portions, and said first structure is said second portion of said at least one deformable retainer.
14. A surgical clip according to item 11, wherein:
   said first structure includes one of inwardly-directed and outwardly-directed projection on at least one of said arms.
15. A surgical clip according to item 11, further comprising:
   e) a prong extending parallel to and between said first and second arms,
      wherein said first and second arms, said prong and said bridge are together configured in an E-shape.
16. A surgical clip according to item 15, further comprising:
   f) at least one deformable retainer extending from one of said arms, wherein said arms are relatively stiffer than said at least one deformable retainer.
17. A surgical clip, comprising:
   a) a first arm having a first width;
   b) a first deformable retainer extending from said first arm, and having a second width less than said first width;
   c) a first catch defined on said first arm adjacent said first retainer;
   d) a second arm having substantially said first width;
   e) a second deformable retainer extending from said second arm, and having a third width less than said first width,
   f) a second catch defined on said second arm adjacent said second retainer,
      wherein said second and third widths have a combined dimension not exceeding said first width; and
   g) a bridge connecting said first and second arms such that said first and second arms extend in substantially a same direction.
18. A surgical clip according to item 17, wherein:
   said second and third widths are each approximately one-half said first width.
19. A surgical clip, comprising:
   a) a first arm;
   b) a first deformable retainer extending from said first arm;
   c) a first catch defined on said first arm adjacent said first retainer;
   d) a second arm;
   e) a second deformable retainer extending from said second arm, said second retainer being horizontally offset relative to said first retainer;
   f) a second catch defined on said second arm adjacent said second retainer; and
   g) a bridge connecting said first and second arms such that said first and second arms extend in substantially a same direction.

## Claims

1. A surgical clip (106, 210, 310, 410, 510, 610, 710, 810, 910) for insertion into tissue, comprising:
a first arm (212, 312, 412, 512, 612, 712, 812, 912);
a second arm (214, 314, 414, 514, 614, 714, 814, 914);
a bridge (216, 316, 416) connecting said first and second arms to form, together, a substantially U-shaped structure having a longitudinal axis, said first and second arms extending in substantially a same direction; and
a surgical structure (232, 540, 640, 740, 760, 762, 860, 862, 931, 932) adapted to at least one of:
prevent rotation of said clip about a longitudinal axis of said clip after said clip is applied to said tissue; and
substantially prevent movement of said arms after said U-shaped structure is applied to the tissue.

2. The surgical clip according to claim 1, comprising a prong (540, 640, 740) extending parallel to and between said first and second arms and having portions (542) projecting substantially perpendicular to said longitudinal axis, wherein said first and second arms, said prong and said bridge are together configured in an E-shape.

3. The surgical clip according to any of the preceding claims, comprising at least one deformable retainer (222, 320, 322, 420, 422, 622, 624, 722, 724, 922, 924) extending from one of said arms, wherein said arms are relatively stiffer than said at least one deformable retainer.

4. The surgical clip according to claim 3, wherein:
said at least one deformable retainer (622, 624) is bifurcated into first (652) and second (654) portions; and
said surgical structure is said second portion of said at least one deformable retainer.

5. The surgical clip according to any of the preceding claims, wherein said surgical structure extends from at least one of said first and second arms.

6. The surgical clip according to any of the preceding claims, wherein:
said first arm has a first distal end (222, 320, 622, 922) and a first tissue piercing portion (224, 321, 654) at said first distal end; and
said second arm has a second distal end (322, 624, 924) and a second tissue piercing portion (323, 658, 925) at said second distal end.

7. The surgical clip according to any of the preceding claims, wherein at least one of said first and second arms have a piercing portion with a distal tip having a first side (924), a second side (925) opposite said first side, and an end surface connecting said first and second sides at an angle.

8. The surgical clip according to any of the preceding claims, wherein said surgical structure (232, 760, 762, 860, 862, 931, 932) projects perpendicular to said longitudinal axis.

9. The surgical clip according to any of the preceding claims, wherein said surgical structure is capable of substantially preventing at least one of:
movement of said arms in a direction perpendicular to said longitudinal axis after said U-shaped structure is applied to the living tissue;
movement of said arms about said longitudinal axis after said U-shaped structure is applied to the living tissue; and
removal of said arms from the living tissue after said U-shaped structure is applied to the living tissue.

10. A surgical clip applier (10), comprising:
a hollow member (12) having a proximal end (14) and a distal end (16);
a clevis (26) coupled to said distal end of said hollow member;
a first jaw (22, 22', 22") rotatably coupled to said clevis;
a second jaw (24, 24', 24") rotatably coupled to said clevis in opposed relation to said first jaw, said first and second jaws adapted to apply a surgical clip (106, 210, 310, 410, 510, 610, 710, 810, 910);
at least one pull/push wire (58, 60) coupled to said first and second jaws and extending through said hollow member to said proximal end of said hollow member; and
an actuation assembly (20) coupled to said proximal end of said hollow member and said proximal end of said at least one pull/push wire for moving said at least one pull/push wire through said hollow member to cause a rotation of said first and second jaws about said clevis from an open to a closed position.

11. The surgical clip applier according to claim 10, wherein at least one of said jaws is provided with a plurality of teeth (50, 50', 50", 52, 52', 52") arranged to puncture and damage tissue adjacent the surgical clip.

12. The surgical clip applier according to either of claims 10 and 11, wherein each of said jaws has:
a clip guiding channel (54, 54', 54", 56, 56', 56") having a channel end; and
a hook-shaped anvil (46, 46', 48, 48', 48") at said channel end.

13. A surgical instrument (100), comprising:
a hollow member (12) having a proximal end (14) and a distal end (16);
a clevis (26) coupled to said distal end of said hollow member;
a first end effector (18) rotatably coupled to said clevis (26);
a first pull/push wire (58, 60) extending through said hollow member to said proximal end of said hollow member;
a first linkage (62, 64, 66, 68) including a first element (62, 66) rotatably coupled to said clevis and coupled to said first pull/push wire, and a second element (64, 68) rotatably coupled to said first element and to said first end effector, said first linkage providing mechanical advantage in rotating said first end effector; and
an actuation assembly (20) coupled to said proximal end of said hollow member and said proximal end of said first pull/push wire for moving said first pull/push wire through said hollow member to cause a rotation of said first end effector about said clevis.

14. The surgical instrument according to claim 13, wherein said first element is a substantially L-shaped member having an elbow rotatably coupled to said clevis.

15. The surgical instrument according to claim 13, comprising:
a second end effector (18) rotatably coupled to said clevis (26) and in opposed relation to said first end effector;
a second pull/push wire (58, 60) extending through said hollow member to said proximal end of said hollow member; and
a second linkage (62, 64, 66, 68) including third (62, 66) and fourth (64, 68) elements, said third element rotatably coupled to said clevis and coupled to said second pull/push wire, said fourth element rotatably coupled to said third element and to said second end effector for increasing mechanical advantage of end effector closure, wherein said actuation assembly is coupled to said second pull/push wire for moving said second pull/push wire through said hollow member to cause a rotation of said second end effector about said clevis.

16. The surgical instrument according to claim 15, wherein said first element and said third element each comprise a substantially L-shaped member having an elbow rotatably coupled to said clevis.

17. A surgical clip application assembly, comprising:
a surgical clip (106, 210, 310, 410, 510, 610, 710, 810, 910); and
a surgical instrument (100) having:
a hollow member (12) having a proximal end (14) and a distal end (16);
a clevis (26) coupled to said distal end of said hollow member;
an end effector (18) rotatably coupled to said clevis and adapted to at least one of:
apply said surgical clip to a location; and
grasp and manipulate tissue;
a first pull/push wire (58, 60) extending through said hollow member to said proximal end of said hollow member;
a dual linkage (62, 64, 66, 68) having:
a first element (62, 66) rotatably coupled to said clevis and coupled to said first pull/push wire; and
a second element (64, 68) rotatably coupled to said first element and to said first end effector, said dual linkage providing mechanical advantage in rotating said first end effector; and
an actuation assembly (20) coupled to said proximal end of said hollow member and to said proximal end of said first pull/push wire for moving said pull/push wire through said hollow member to cause a rotation of said first end effector about said clevis.

18. The surgical clip application assembly according to claim 17, wherein:
said end effector (18) has first (22, 22', 22") and second (24, 24', 24") jaws pivotally coupled to said clevis (26) for applying said surgical clip (106, 210, 310, 410, 510, 610, 710, 810, 910) to the location;
said dual linkage (62, 64, 66, 68) is a first dual linkage;
said first jaw is coupled to said second element (64, 68) of said first dual linkage and pivots at said clevis when said actuation assembly is actuated; and
further comprising:
a second pull/push wire (58, 60) extending through said hollow member and operatively coupled to one of said first pull/push wire and said actuation assembly (20) for moving said second pull/push wire through said hollow member to cause a rotation of said second jaw about said clevis; and
a second dual linkage (62, 64, 66, 68) having:
a third element (62, 66) rotatably coupled to said clevis and coupled to said second pull/push wire; and
a fourth element (64, 68) rotatably coupled to said third element and rotatably coupled to said second jaw such that said second jaw pivots at said clevis when said actuation assembly is actuated.

19. The surgical clip application assembly according to claim 18, wherein:
said first and third elements are L-shaped having an elbow rotatably coupled to said clevis; and
said first and third elements are coupled to a respective one of said pull/push wires proximal of said elbow.

20. The surgical clip application assembly according to claim 18, wherein:
at least one of said first and second jaws has:
a clip guiding channel (54, 54', 54", 56, 56', 56") shaped to fit said surgical clip (106, 210, 310, 410, 510, 610, 710, 810, 910), said clip guiding channel having an end; and
a hook-shaped anvil (46, 46', 48, 48', 48") at said end of said clip guiding channel; and
said first and second jaws apply said surgical clip with said clip guiding channel.
